# EUROPEAN PATENT SPECIFICATION

(11) **EP 2 101 796 B1**
(45) Date of publication and mention of the grant of the patent: **16.11.2011**
(21) Application number: 07817985.0
(22) Date of filing: 08.10.2007
(51) Int. Cl.: A61K 35/74, C12N 1/20

(54) **BACILLUS SMITHII STRAIN TBMI12 MSCL P737 AND USE OF ENDOSPORES THEREOF AS A PROBIOTIC OR A FOOD SUPPLEMENT**
BACILLUS SMITHII-STAMM TBMI12 MSCL P737 UND VERWENDUNG VON ENDOSPOREN DAVON ALS PROBIOTISCHES MITTEL ODER NAHRUNGSZUSATZ
SOUCHE DE BACILLUS SMITHII TBMI12 MSCL P737 ET UTILISATION D'ENDOSPORES DE CELLE-CI EN TANT QUE PROBIOTIQUE OU SUPPLÉMENT ALIMENTAIRE

(30) Priority: 08.12.2006 EE 200600038
(43) Date of publication of application: 23.09.2009
(73) Proprietor: Tartu Ülikool (University Of Tartu), 50090 Tartu (EE)
(72) Inventor: JÖGI, Eerik, 61401 Tartumaa (EE); NURK, Allan, 50105 Tartu (EE); SUITSO, Indrek, 50414 Tartu (EE); TALPSEP, Ene, 51011 Tartu (EE); NAABER, Paul, 50108 Tartu (EE); LÕIVUKENE, Krista, 50410 Tartu (EE)
(74) Representative: Kahu, Sirje
(86) International application number: PCT/EE2007/000021
(87) International publication number: WO 2008/067827

(56) References cited:
- SUITSO ET AL: "P1763 Bacillus smithii TBMI12 spores as a potential competitive exclusion agent against Salmonella enteritidis" INTERNATIONAL JOURNAL OF ANTIMICROBIAL AGENTS, AMSTERDAM, NL, vol. 29, March 2007 (2007-03), page S501, XP022038952 ISSN: 0924-8579
- JOGI E., TALPESP E.: "Bacillus smithii strain TBMI12 16S ribosomal RNA gene, partial sequence"[Online] 25 October 2006 (2006-10-25), XP002465105 Retrieved from the Internet: URL:http://srs.ebi.ac.uk/srsbin/cgi-bin/wg etz?-e+([emblidacc-id:EF010852]>embl) [emb l-acc:EF010852]+-vn+2+-noSession> [retrieved on 2008-01-18]
- MIYASHITA M., NAKAGAWA Y., SUZUKI K.: "Bacillus smithii gene for 16S rRNA, partial sequence"[Online] 5 September 2006 (2006-09-05), XP002465106 Retrieved from the Internet: URL:http://srs.ebi.ac.uk/srsbin/cgi-bin/wg etz?-e+([emblidacc-id:AB271749]>embl) [emb l-acc:AB271749]+-vn+2+-noSession> [retrieved on 2008-01-18]
- SCHELDEMAN PATSY ET AL: "Incidence and diversity of potentially highly heat-resistant spores isolated at dairy farms." APPLIED AND ENVIRONMENTAL MICROBIOLOGY MAR 2005, vol. 71, no. 3, March 2005 (2005-03), - March 2005 (2005-03) pages 1480-1494, XP002465107 ISSN: 0099-2240 & [Online] 2005, Retrieved from the Internet: URL:http://srs.ebi.ac.uk/srsbin/cgi-bin/wg etz?-e+([emblidacc-id:AY373319]>embl) [emb l-acc:AY373319]+-vn+2+-noSession> [retrieved on 2008-01-18]
- RAINEY F A ET AL: "THE PHYLOGENETIC DIVERSITY OF THERMOPHILIC MEMBERS OF THE GENUS BACILLUS AS REVEALED BY 16S RDNA ANALYSIS" FEMS MICROBIOLOGY LETTERS, AMSTERDAM, NL, 1994, pages 205-211, XP002921596 ISSN: 0378-1097 & [Online] 14 May 1997 (1997-05-14), Retrieved from the Internet: URL:http://srs.ebi.ac.uk/srsbin/cgi-bin/wg etz?-e+([emblidacc-id:Z26935]>embl) [embl- acc:Z26935]+-vn+2+-noSession> [retrieved on 2008-01-18]

## Description

### FIELD OF THE INVENTION

The invention belongs to the field of biotechnology and relates to a probiotic bacterial strain that can be used to prevent bacterial infections of gastrointestinal tract of vertebrate animals and to stimulate their immune systems.

### BACKGROUND OF THE INVENTION

The idea to use the antagonism between bacteria against pathogens dates back to the times of Lister and Fleming. Administering living microbes to colonize the gastrointestinal tract, to stimulate the immune system, or for antibacterial purposes is a much more recent idea. Orally administered living microorganisms that benefit the organism who consumes them are called probiotics (Martins, F. S. et al. (2005) Screening of yeast as probiotic based on capacities to colonize the gastrointestinal tract and to protect against enteropathogen challenge in mice. J. Gen. Appl. Microbiol. 51: 83-92). Traditionally, these originate from the genus *Lactobacillus.*
Unfortunately, the successful use of lactobacilli as probiotics is hindered by the massive perishing of microbes due to gastric acid and bile (Spinosa, M.R. et al. (2002) On the fate of ingested Bacillus spores. Res. Microbiol. 151: 361-368). However, if spores are used instead of vegetative cells, their survival rate is considerably higher. At the same time, preparations administered in the form of spores are not less effective than preparations administered in the vegetative form. Rather, the possibility to preserve spores for an unlimited period of time is an advantage and thus the administering of spores has become an alternative to using antibiotics in agriculture (Wolken, W.A.M. et al. (2003) What can spores do for us? Trends in Biotechnology. 21: 338-345). Spores of the representatives of the genus *Bacillus,* such as *B. clausii, B*. *subtilis, B. pumilus* and *B. cereus* are mostly used for prophylactic and therapeutic purposes (Duc, L.H. et al. (2004) Characterization of Bacillus probiotics available for human use. Appl. Environ Microbiol. 70: 2161-2171).

What has aroused interest is the possibility to use a combination of *Bacillus* sp. endospores and antibiotics to fight infections. This practice is common in East and South Asia, since it reduces the complications of antibiotic treatment.
As a rule, the used endospores are resistant to the relevant antibiotic as well. A disadvantage of such a technique is the spreading of antibiotic resistance genes.

The closest prior art of the current invention involves the sporogenous bacterium *Bacillus coagulans,* which is used as a probiotic additive in animal feedstuffs (WO 93/14187, Adami A. et al., 1993), and a combination of probiotic non-sporogenous *Bacillus subtilis* and/or *Bacillus clausii* microorganisms and spores for pharmaceutical, dietary and/or nutraceutical use (EP1405641A2, Dondi G., 2004). These experiments are usually indirect: the probiotic effect is tested primitively *in vitro* (EP 1107772, Farmer, Sean. Probiotic, lactic acid-producing bacteria and uses thereof 1999) or the positive weight gain of test animals is simply measured (Adami, A., et al. (1993) A bacterial strain of the species *Bacillus coagulans:* its use as a probiotic agent. PCT/EP93/0030).

### DISCLOSURE OF INVENTION

The subjects of the present invention are the strain of microorganism *Bacillus smithii* TBMI12 MSCL P737 , and the composition comprising the said strain, composition for use, as a probiotic for antibacterial purposes, for colonizing the gastrointestinal tract and for stimulating the immune system, as well as use of said strain as a food supplement and as a component in therapeutic and non-therapeutic the probiotic composition.

The subject of the current invention - *Bacillus smithii* TBMI12 MSCL P737 - was isolated from the faeces of a healthy adult man. The obtained strain was isolated in the process of describing the microbiological composition of the faeces in which the existence of thermophilic *Bacillus* sp. species was tested. The fresh faecal sample was diluted ten-fold in 0.9% NaCl solution and suspended. The obtained suspension was heated at 85°C for 15 minutes to eliminate vegetative cells. The heated sample was cultivated on a medium with the composition specified in (Michelson, T.; Kask, K.; Jðgi, E.; Talpsep, E.; Suitso, I.; Nurk, A. (2006). 1(+)-Lactic acid producer Bacillus coagulans SIM-7 DSM 14043 and its comparison with Lactobacillus delbrueckii ssp. lactis DSM 20073. Enzyme and Microbial Tech). The plates with the inoculation rate 100 *µ*l were incubated at 55°C for 24 h. A suspension (0.2% Tween80) of different dilutions was made of the obtained colonies and by cultivating it on the above-mentioned medium, a purified pure culture of *Bacillus smithii* TBMI12 MSCL P737 was obtained.

**Culture and morphological characteristics**. Colonies of the strain *Bacillus smithii* TBMI12 MSCL P737 have a straight edge, convex surface and very slimy consistence. The diameter of colourless (without pigment) colonies is approx. 5 mm. Gram-positive rod-shaped bacteria are usually unagregated, but in case of stress also form chains. Cells are motile and form subterminal endospores.

**Physiological and biochemical characteristics.** The strain *Bacillus smithii* TBMI12 MSCL P737 grows on monosaccharides such as glucose, galactose, fructose, xylose and arabinose; on disaccharides such as sucrose, maltose, lactose; and on polysaccharides such as starch. As to the type of metabolism, the strain is an aerotolerant lactic acid bacterium: it ferments carbohydrates into lactic acid without releasing carbon dioxide. Due to the existence of catalase it is tolerant to oxygen.

**Growth temperature.** The strain *Bacillus smithii* TBMI12 MSCL P737 grows at a wide range of temperatures from room temperature to 55°C. 40 minutes of heating at 85 °C does not harm endospores or influence their germination.

**Identification.** The strain *Bacillus smithii* TBMI12 MSCL P737 was identified on the basis of the 16S rRNA gene sequence (GenBank Accession No EF010852). The closest relatives were *Bacillus smithii* R-71170 with a 99% similarity, *Bacillus smithii* JCM9076 with a 96% similarity and *Bacillus* eolicus 4-1T with a 95% similarity.

Since 19.10.2006, the strain *Bacillus smithii* TBMI12 MSCL P737 is deposited in the Microbial Strain Collection of Latvia (MSCL) under the number P737.

*Bacillus smithii* TBMI12 MSCL P737 provides an interesting opportunity, as due to its type of metabolism the strain is resistant to metronidazole. This makes it possible to use the strain *Bacillus smithii* TBMI12 MSCL P737 without additional resistance markers in the treatment of gastrointestinal tract infections induced by *Clostridium difficile,* since the above-mentioned *Clostridium difficile* is sensitive to metronidazole and this antibiotic is usually used for treatment. In addition to this antibiotic, it is in principle possible to use other antibiotics together with the strain *Bacillus smithii* TBMI12 MSCL P737.

The strain according to the current invention is used for the following purposes.
Colonization of the gastrointestinal tract for antibacterial purposes.
*Clostridium difficile,* which is part of the normal microflora of humans, is generally harmless. However, it may cause pseudomembraneous colitis after antibiotic treatment that impairs the microflora balance of the gastrointestinal tract. To avoid the proliferation of *Clostridium difficile* and consequent sickness, the gastrointestinal tract should preventively be colonized with a probiotic bacterium, such as the strain *Bacillus smithii.* TBMI12 MSCL P737, which can survive antibiotic treatment in the form of endospores.

Colonization of the gastrointestinal tract in order to provide resistance to disease and prevent bacterial infections.
*Salmonella* infection is usually food-borne. Bacteria that have entered the gastrointestinal tract attach to the intestine wall, penetrate it and are carried all over the body by blood. In the liver the bacteria replicate and afterwards move back to the intestine where they induce diarrhoea. Diseased domestic animals and birds may infect people through eggs and meat.

Although the disease is usually not life-threatening to humans (only 2 % of infected people need antibiotic treatment), the preventive colonization of the gastrointestinal tract of humans, domestic animals and birds with the strain *Bacillus smithii* TBMI12 MSCL P737 is more reasonable than getting sick.

### Use for the purpose of stimulating the immune system.

Keeping animals in hygienic conditions reduces the risk of pathogen infections. Unfortunately, this also has a negative side effect: the immune system becomes used to sterile conditions and even accidental contact with a usually non-pathogenic microorganism may cause sickness. To avoid this, the immune system should constantly be exposed to immunogens.
Administration of endospores of the probiotic bacteria *Bacillus smithii* TBMI12 MSCL P737 enables to keep the immune system constantly ready to fight against pathogens.

### Use as a food supplement.

The strain *Bacillus smithii* TBMI12 MSCL P737 can be used as a food supplement in the form of both vegetative cells and endospores. Vegetative cells must be lyophilized before administration. Administration of the strain *Bacillus smithii* TBMI12 MSCL P737 in the form of endospores is preferred as it enables to achieve the maximum probiotic effect and, at the same time,
endospores can be preserved easily. The use of endospores in the form of dry preparations is preferred.
In such a case, there are basically no limitations also to the excipients of the preparation, since cells in an inactive form such as endospores tolerate extreme conditions (high sugar concentration, low pH, long-term preservation, preservatives).

### Use as a component of probiotic compositions.

Vegetative cells or endospores of *Bacillus smithii* TBMI12 MSCL P737 can be used as a component in probiotic compositions. The function of excipients is to facilitate production, preservation and administration, as well as to support the probiotic effect of *Bacillus smithii* TBMI12 MSCL P737.

### External use for antibacterial purposes.

The antagonism between bacteria can be used to hygienize the external surface of the host organism from harmful bacteria. What is noteworthy is the fact that differently from chemical or physical sterilization that unselectively destroys the whole microflora, the antagonism between bacteria functions selectively. This means that it is possible to suppress potential pathogens without disturbing the normal microflora.

### BRIEF DESCRIPTION OF DRAWINGS

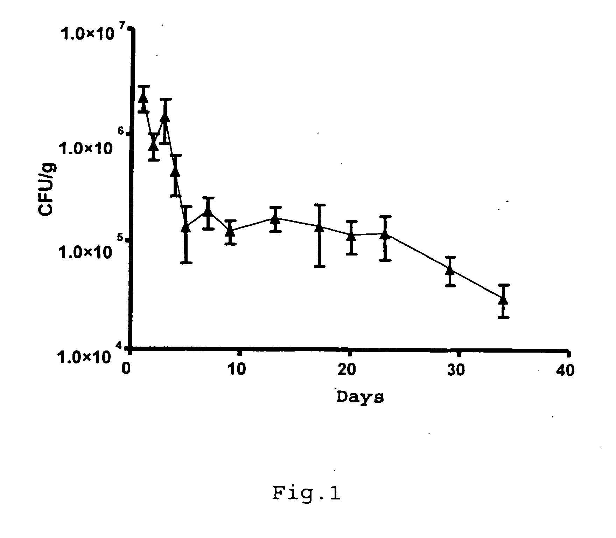

Fig.1. The number (■) of *Bacillus smithii* TBMI12 MSCL P737 (n=5) with standard error in the faeces of hamsters (CFU/g) after antibiotic treatment (ampicillin 3 mg/hamster), inoculation with *Clostridium difficile* VPI 10463 (10⁴ cells), and treatment with the probiotic strain *Bacillus smithii* TBMI12 MSCL P737 (1 x 10⁸ endospores; on the 2nd, 3rd and 4th day).

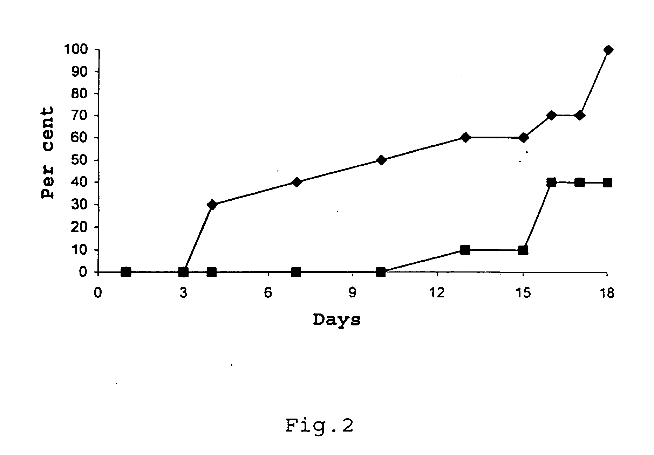

Fig.2. Course of *Salmonella* infection in mice. Infection with the pathogen *Salmonella* Enteritidis wt in mice not colonized (■) and those colonized (■) with *Bacillus smithii* TBMI12 MSCL P737 endospores was detected from samples of faeces, liver or spleen cultivated on an XLD plate according to CFU.

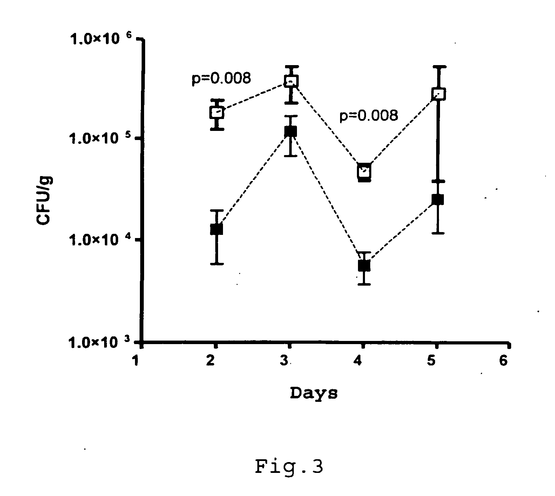

Fig.3. Median values of the number of *Clostridium diffi*cile VPI 10463 with standard error from the faeces of the test animals (CFU/g). The mice who received combined treatment with metronidazole + *Bacillus smithii* TBMI12 MSCL P737 (10⁸) (n=5) (■) and the mice treated only with metronidazole (■). The value p on the 2nd and 4th day indicates the statistical significance of the difference.

### DESCRIPTION OF EMBODIMENT

### 1. Colonization of the gastrointestinal tract for antibacterial purposes.

In order to demonstrate in an *in vivo* experiment the ability of *Bacillus smithii* TBMI12 MSCL P737 to colonize successfully the gastrointestinal tract of vertebrate animals, we used hamsters (*Mesocritus auratus*) as test animals.
The microflora of normal hamsters was impaired by 3 mg of intragastrically administered ampicillin. After 24 hours the test animals were infected with cells (à 10⁵) of *Clostridium difficile* strain VPI 10463. Half of the hamsters were administered *Bacillus smithii* TBMI12 MSCL P737 endospores (à 10⁸) two hours before infection. In addition, the same dose was administered to the same group of hamsters 24 and 48 hours later. Hamsters who were not administered and thus not colonized with the strain *Bacillus smithii* TBMI12 MSCL P737 died in 48 hours, while the colonized hamsters survived. *Bacillus smithii* TBMI12 MSCL P737 still colonized the gastrointestinal tract during one month after the administration (Fig.1). The result is particularly valuable as hamsters are especially vulnerable to the toxin of *Clostridium difficile* and this bacterium (*Bacillus smithii* TBMI12 MSCL P737) is not part of their normal microflora.
With this experiment we demonstrated that *Bacillus smithii* TBMI12 MSCL P737 is capable of colonizing the gastrointestinal tract for a long period and thus prevent *Clostridium difficile* infection in hamsters.

### 2. Colonization of the gastrointestinal tract in order to provide resistance to disease and prevent bacterial infections

For persistent colonization of the gastrointestinal tract of mice (*Mus musculus* BALB/c), three doses (à 10⁸) of endospores of the probiotic bacterium *Bacillus smithii* TBMI12 MSCL P737 were intragastrically administered in every 24 hours. On the third day after the last dose the test animals were inoculated with *Salmonella* Enteritidis wt cells (à 10⁶). Two weeks later it was possible to cultivate *Salmonella* Enteritidis cells from the samples of liver, spleen and faeces of only 40% of the test animals. 60% of the test animals were not infected with *Salmonella.* The control group was not colonized with the strain *Bacillus smithii* TBMI12 MSCL P737 and only *Salmonella* Enteritidis wt cells (à 10⁶) were administered. All of these mice were infected. The course of the disease was considerably slowed down: infected mice who had been colonized with the strain *Bacillus smithii* TBMI12 MSCL P737 showed symptoms only on the 10th day after infection, while it was possible to detect *Salmonella* Enteritidis cells in the faeces of uncolonized mice already on the first day after the infection. The experiment described above is illustrated by Fig.2. Due to the slower course of the disease it is possible to take measures to fight against infection and to ease the pathological condition. It is possible to conclude on the basis of the experiments that the use of the strain *Bacillus smithii* TBMI12 MSCL P737 as a probiotic agent enables to prevent *Salmonella* infection and to slow down the course of the disease.

### 3. Use for the purpose of stimulating the immune system

In order to stimulate the immune system of mice, three doses (à 10⁸) of endospores of the strain *Bacillus smithii* TBMI12 MSCL P737 were administered intragastrically every 24 hours. Four hours after the last dose the test animals were infected with *Salmonella* Enteritidis wt cells (à 10⁶). Due to the prior stimulation of the immune system, the test animals were not infected with *Salmonella.* It was possible to detect *Salmonella* Enteritidis cells for the first time only on the 10th day after inoculation and in the faeces of only 10% of the test animals. In total, only 40% of test animals colonized with *Bacillus smithii* TBMI12 MSCL P737 endospores were infected with *Salmonella.* In the control group the immune systems of mice were not stimulated with endospores of the strain *Bacillus smithii* TBMI12 MSCL P737 and only *Salmonella* Enteritidis wt cells (à 10⁶) were administered. 100% of these mice were infected. The course of the disease accelerated as well: it was possible to detect *Salmonella* Enteritidis cells in 30% of the faeces of uncolonized mice already on the first day after inoculation. Accordingly, repeated administration of endospores of the probiotic bacterium strain *Bacillus smithii* TBMI12 MSCL P737 stimulates the immune system sufficiently to provide significant protection against *Salmonella* infection.

### 4. Use as a food and/or feed supplement

Mice and hamsters were fed *Bacillus smithii* TBMI12 MSCL P737 endospores in different concentrations and their effect on the test animals was observed. No harmful effect appeared. At the same time, the positive results were evident: the test animals' microflora that had been altered with antibiotic treatment could be restored and the test animals were much more resistant to bacterial infections of the gastrointestinal tract, since *Bacillus smithii* TBMI12 MSCL P737 endospores had stimulated their immune systems beforehand.
Accordingly, the probiotic bacterium strain *Bacillus smithii* TBMI12 MSCL P737 is suitable for using as a food and/or feed supplement.

### 5. Use as a component of probiotic compositions

In order to administer mice and hamsters *Bacillus smithii* TBMI12 MSCL P737 endospores, a probiotic composition was made of the latter and water deionized through reverse osmosis. The probiotic composition contained 10⁶ to 10¹⁰ endospores per 1 ml of solution. This facilitated considerably the preservation and administration issues. Due to the administration of the composition containing the strain *Bacillus smithii* TBMI12 MSCL P737, the test animals' resistance to bacterial infections of the gastrointestinal tract was increased. Accordingly, the probiotic bacterium strain *Bacillus smithii* TBMI12 MSCL P737 is suitable for using as the component of probiotic compositions.

### 6. External use for antibacterial purposes

To demonstrate the antibiosis between *Bacillus smithii* TBMI12 MSCL P737 and other bacteria, *in vitro* experiments were performed. At first, *Bacillus smithii* TBMI12 MSCL P737 cells were grown on PDA (Fluka) plates at 55°C and subsequently bacteria of different species were cultivated radially around them. The experiment involved

*Bacillus cereus, Aeromonas hydrophila, Erwinia carotovora, Pseudomonas fluorescens, Serratia marcesens, Enterobacter aerogenes, Escherichia coli, Micrococcus luteus, Staphylococcus aureus, Proteus vulgaris, Salmonella* Typhimurium and *Salmonella* Enteritidis. Thereafter the plates were incubated at 30°C. 75% of the bacterial species involved in the experiment showed growth inhibition near the *Bacillus smithii* TBMI12 MSCL P737 colony. It should be noted that those species included several pathogens, such as *Salmonella* Enteritidis, *Salmonella* Typhimurium, *Staphylococcus aureus.* At the same time, the 25% whose growth was not inhibited by *Bacillus smithii* TBMI12 MSCL P737 included species of the normal microflora, such as *Escherichia coli.*
Accordingly, there is antibiosis performed between *Bacillus smithii* TBMI12 MSCL P737 and other bacteria, which makes it possible to use *Bacillus smithii* TBMI12 MSCL P737 externally for antibacterial purposes.

### 7. Use of a probiotic composition in interaction with antibiotics

To demonstrate the positive interaction of *Bacillus smithii* TBMI12 MSCL P737 endospores with antibiotics we performed an *in vivo* experiment with mice (BALB/c). Before infection with the toxic strain *Clostridum difficile* VPI 10643 the mice needed a 5-day treatment with cefoxitin to weaken the microflora of the test animals. After inoculation with the strain *Clostridum difficile* VPI 10643 the mice were divided into two groups. The first group of mice was administered metronidazole to treat *Clostridium difficile* (24 h after inoculation; 15 mg/kg), and the other group of mice was administered *Bacillus smithii* TBMI12 MSCL P737 endospores (10⁸ endospores with each dose of antibiotics 15 mg/kg) in addition to metronidazole.
The experiment revealed that in the case of mice who received combined treatment (metronidazole + *Bacillus smithii* TBMI12 MSCL P737), the number of *Clostridium difficile* in faeces was reduced more than 10 times (Fig.3). Thus the administration of *Bacillus smithii* TBMI12 MSCL P737 endospores has potential for use in antibiotic treatment.

The uses of this invention are not limited to the examples described herein; other embodiments are possible as well.

## Claims

1. The strain of microorganism *Bacillus smithii* TBMI12 MSCL P737.

2. Use of the strain of microorganism of Claim 1 as a food and/or feed supplement.

3. The strain of microorganism of Claim 1 as a component for use in therapeutic probiotic compositions.

4. Use of the strain of microorganism of Claim 1 as a component in non-therapeutic probiotic compositions.

5. A composition comprising the the strain of microorganism of Claim 1.

6. A composition of Claim 5, further comprising one or more antibiotics.

7. A composition of Claim 6, wherein the named antibiotic is metronidazole.

8. Composition of Claim 5 for use as a probiotic for the purpose of colonizing the gastrointestinal tract.

9. Composition of Claim 5 for use as a probiotic for the purpose of preventing bacterial infections of the gastrointestinal tract.

10. Composition of Claim 5 for use as a probiotic to stimulate the immune system.

11. Composition of Claim 5 for external use as a medicine for antibacterial purposes.

12. Composition of Claim 5 for use as a medicine to reduce or eliminate deficiencies and side effects caused by antibiotic treatment.

## Patentansprüche

1. Der Stamm des Mikroorganismus *Bacillus smithi* TBMI12 MSCL P737.

2. Verwendung des Stammes des Mikroorganismus nach Anspruch 1 als Nahrungs-und/oder Futterzusatz.

3. Verwendung des Stammes des Mikroorganismus nach Anspruch 1 als Bestandteil von therapeutischen probiotischen Kompositionen.

4. Verwendung des Stammes des Mikroorganismus nach Anspruch 1 als Bestandteil von nichttherapeutischen probiotischen Kompositionen.

5. Eine Komposition, die den Stamm des Mikroorganismus nach Anspruch 1 enthält.

6. Eine Komposition nach Anspruch 5, die zusätzlich ein oder mehrere Antibiotika enthält.

7. Eine Komposition nach Anspruch 6, wobei das angegebene Antibiotikum Metronidazol ist.

8. Eine Komposition nach Anspruch 5, die als Probiotikum zur Kolonisierung des Verdauungstraktes verwendet wird.

9. Eine Komposition nach Anspruch 5, die als Probiotikum zur Vorbeugung bakterieller Infektionen des Verdauungstraktes verwendet wird.

10. Eine Komposition nach Anspruch 5, die als Probiotikum zur Stimulierung des Immunsystems verwendet wird.

11. Eine Komposition nach Anspruch 5, die als äußerlich anzuwendendes antibakterielles Arzneimittel verwendet wird.

12. Eine Komposition nach Anspruch 5, die als Arzneimittel zur Verringerung oder Beseitigung von Mängeln und Nebenerscheinungen verwendet wird, die bei der Antibiotikumtherapie auftreten.

## Revendications

1. La souche de microorganisme Bacillus smithii T13MI12 MSCL P737.

2. L'utilisation de la souche de microorganisme de la revendication 1 comme aliment et / ou complément alimentaire.

3. La souche de microorganisme de la revendication 1 comme composant pour l'utilisation dans des compositions thérapeutiques probiotiques.

4. L'utilisation de la souche du microorganisme de la revendication 1 comme composant des compositions non thérapeutiques probiotiques.

5. La composition qui comprend la souche du microorganisme de la revendication 1.

6. La composition de la revendication 5 qui comprend en outre un ou plusieurs antibiotiques.

7. La composition de la revendication 6, selon laquelle l'antibiotique en question est le métronidazole.

8. Composition de la revendication 5, utilisée comme probiotique au but de coloniser le tractus gastro-intestinal.

9. Composition de la revendication 5, utilisée comme probiotique au but de prévenir les infections bactériennes du tractus gastro-intestinal.

10. Composition de la revendication 5, utilisée comme probiotique pour stimuler le système immunitaire.

11. Composition de la revendication 5 pour l'usage externe comme un médicament à des fins anti-bactériennes.

12. Composition de la revendication 5, utilisée comme médicament pour réduire ou éliminer les déficiences et les effets secondaires causés par le traitement antibiotique.
